# EUROPEAN PATENT APPLICATION

(11) **EP 1 764 088 A1**
(43) Date of publication of application: **21.03.2007**
(21) Application number: 06394016.7
(22) Date of filing: 23.08.2006
(51) Int. Cl.: A61K 9/06, A61K 31/737, A61K 47/36

(54) **Thixotropic personal lubricant containing carageenan**

(30) Priority: 24.08.2005 US 210293; 09.08.2006 US 501609
(71) Applicant: Roman, Stephen B., Phoenix, Arizona 85020 (US); Kehoe, Gary S., Phoenix 85020 (US)
(72) Inventor: Roman, Stephen B., Phoenix, Arizona 85020 (US); Kehoe, Gary S., Phoenix 85020 (US)
(74) Representative: McCarthy, Denis Alexis

(57) **Abstract**

A thixotropic personal lubricant includes a high molecular weight, thixotropic, gel-forming, naturally occurring polysaccharide extracted from algae and comprised of repeating sulfated and non-sulfated galactose and 3,6 anhydrogalactose (3,6-AG) units, and includes water.

## Description

This invention pertains to personal lubricants for epithelial tissue layers, especially mucosal tissue, which lubricants can provide high lubricity, provide protection from disease, deliver medicaments, and exhibit a viscosal stability sufficient to maintain the personal lubricants in contact with mucousal tissue for extended periods of time. The invention also pertains to methods of preparing, dispensing and using personal lubricants in accordance with the invention.

Conventional personal lubricants, once applied, tend to lose their viscosity and travel over and away from desired mucosal tissues, particularly vaginal and other tissues utilized during sexual intercourse. Consequently, it is difficult to apply such lubricants in advance and to have the lubricants remain in position for an extended period of time. In addition, such lubricants can contain artificial compositions, can permit movement of macrophages through the lubricant, and can be inconvenient to apply.

We have discovered an improved personal lubricant composition. The composition comprises a thixotropic gel having a viscosity in the range of 1,000 to 80,000 centipoise; having a pH in the range of 2.6 to 10; and, comprising naturally occurring compositions. The naturally occurring compositions preferably include a high molecular weight, thixotropic, gel-forming, naturally-occurring polysaccharide extracted from algae and composed of repeating sulfated and non-sulfated galactose and 3,6 anhydrogalactose (3,6-AG) units; and, water.

Another embodiment of the invention comprises a method of lubricating mucosal tissue in the body to facilitate movement of a selected object over the tissue. The method comprises the step of providing a container of a personal lubricant thixotropic gel to dispense multiple equivalent doses of the gel. The gel has a viscosity in the range of 1,000 to 80,000 centipoise, has a pH in the range of 2.6 to 10, and comprises water and a high molecular weight, thixotropic, gel-forming, naturally-occurring polysaccharide extracted from algae and composed of repeating sulfated and non-sulfated galactose and 3,6 anhydrogalactose (3,6-AG) units. The method also includes the steps of applying to the mucosal tissue with the container at least two equivalent doses of the gel; and, moving the selected object over the mucosal tissue and gel to reduce the viscosity of the thixotropic gel and facilitate movement of the select object over the mucosal tissue.

The particular polysaccharide presently utilized in the practice of the invention is critical. Although a multitude of polysaccharides exist, the critical polysaccharide utilized in the invention is a high molecular weight thixotropic polysaccharide made up of repeating galactose and 3,6 anydrogalactose (3,6-AG) units, both sulfated and nonsulfated and extracted from algae, typically Eucheuma, Chondrus, and Gigartina red benthic marine algae that are multicellular and macrothallic. As used herein, a polysaccharide is thixotropic when it produces a thixotropic solution or gel when admixed with water or another liquid.

Three specific types of high molecular weight galactose polysaccharides extracted from marine algae are kappa, iota, and lambda.

Kappa polysaccharide typically forms a strong, rigid aqueous gel; has some syneresis; and, forms a helix with potassium ions. Calcium ions cause the helices in kappa formed gel to aggregate and cause the gel to contract and become brittle. Gel formed with kappa polysaccharide is slightly opaque, but becomes clear when sugar is added. Kappa polysaccharide is about 25% ester sulfate and about 34% 3,6-AG.

lota polysaccharide forms an elastic aqueous thixotropic gel and forms a helix with calcium ions. Limited aggregation in iota formed gel contributes to the elasticity of the gel. There is no syneresis. The gel is clear. When iota formed gel is frozen and thawed, its viscosity remains stable, as generally do gels formed with iota polysaccharide in combination with kappa polysaccharide and/or lambda polysaccharide. Iota polysaccharide is about 32% ester sulfate and 30% 3, 6-AG.

Lambda polysaccharide does not form an aqueous gel. Lambda polysaccharide is about 35% ester sulfate and includes little or no 3,6-AG.

While lambda and kappa polysaccharide can be utilized alone, in combination with each other, or in combination with iota polysaccharide in producing gels utilized in the invention, when a thixotropic gel is desiredBwhich is the case in the presently preferred embodiment of the inventionBiota polysaccharide must be utilized. Iota polysaccharide presently comprises at least 25%, preferably at least 33%, most preferably at least 50% of a quantity of high molecular weight galactose algae polysaccharide utilized to prepare a batch or quantity of personal lubricant in accordance with the invention. The remaining portion of the quantity of galactose algae polysaccharide used to prepare a batch of personal lubricant can comprise lambda or kappa polysaccharide. When solids are admixed with water to produce a person lubricant, the high molecular weight galactose algae polysaccharide comprises at least 50%, preferably at least 75%, most preferably at least 80% of the solids, while water or other liquids comprise the remainder of the composition.

The concentration of high molecular weight galactose polysaccharide in the personal lubricant of the invention is in the range of 0.5% to 5.0% by weight, preferably in the range of 1% to 4% by weight, most preferably in the range of 1.5% to 3.5% by weight. As noted, the galactose polysaccharide can consist of iota, lambda, and/or kappa polysaccharide.

If the personal lubricant of the invention includes 0.5% by weight of iota galactose polysaccharide, thixotropic properties are not apparent. If the lubricant includes 0.75% by weight iota polysaccharide, some thixotropic properties are evidenced. 1.0% by weight of iota polysaccharide provides more evidence of thixotropic properties; 1.5% by weight provides good evidence; and, when there is 1.75% by weight iota polysaccharide the thixotropic property of the gel lubricant is very noticeable. Consequently, it is preferred that the personal lubricant of the invention include at least 1.0% by weight iota polysaccharide, preferably at least 1.5% by weight iota polysaccharide, and most preferably at least 1.75% by weight iota polysaccharide. Lesser fractions of lambda and kappa polysaccharides are normally, but not necessarily, included with iota polysaccharide.

Iota, kappa, and lambda polysaccharides are sold by various sources, including FMC Corporation, 1735 Market Street, Philadelphia, PA 19103, and CP Kelco, 311 S, Wacker Drive, Suite 3700, Chicago, Illinois 60606. Examples of galactose polysaccharides sold by FMC Corporation are 373/Gelcarin GP 911 [Kappa polysaccharides comprise at least majority of composition], 335/Gelcarin GP 379 [lota polysaccharides comprise at least majority of composition], 303/Gelcarin GP 812 [Kappa polysaccharides], 205/Viscarin GP 109 [Lambda polysaccharides], 201/Viscarin GP 209 [Lambda polysaccharides], and, 357/Seaspen PF [Iota polysaccharides, phosphates, CaS04-2H20]. Examples of galactose polysaccharides sold by CP Kelco are Genuvisco type X-931-03 (CP Kelco), and Genuvisco type X-923-03 (CP Kelco) [lota polysaccharides].

Glycerin can be included in the personal lubricant of the invention as an emollient and to slow the evaporation of moisture from the lubricant. Glycerin is a naturally occurring substance and can comprise from 1% to 25% by weight of the personal lubricant. It is presently preferred to incorporate from 5% to 10% by weight glycerin in the personal lubricant. Propylene glycol or any other conventional desired emollients can be utilized in the personal lubricant.

Minor effective amounts of preservatives, typically in the range of 0.01% to 1.0% by weight, can be included in the personal lubricant. By way of example, and not limitation, methylparaben, propylparaben, potassium sorbate, and benzoic acid are common preservatives than can be utilized.

Effective amounts of appropriate acidic or basic compositions can be include in the personal lubricant to adjust and control pH in the desired range of 2.6 to 10. The presently preferred pH is 4.0. By way of example, and not limitation, citric acid and sodium hydroxide comprise compositions commonly utilized to adjust the pH of the personal lubricant.

Minor effective amounts of flavoring, topical stimulants (i.e., to produce a warming or cooling sensation) coloring, or odor producing compositions can be incorporated in the personal lubricant in either a liquid, solid or gaseous form or mixture thereof.

The water utilized preparing the personal lubricant can be de-ionized water, USP water, de-chlorinated water, mineral water, water treated with activated carbon, tap water, etc. Naturally occurring oils or other fluids can, if desired, be utilized in place of or in combination with water.

Dosage can vary per the user's discretion, but the volume of a single dose typically is in the range of 1.0 mL (milliliter) to 15 mL.

The following examples are given by way of illustration and not limitation of the invention.

### EXAMPLE I

The following ingredients are provided.

| Ingredient | Weight Percent |
|---|---|
| lota polysaccharide | 2.0 |
| Kappa polysaccharide | 0.5 |
| Lambda polysaccharide | 0.5 |
| Sodium Hydroxide (pH adjustment) | .05 |
| Methylparaben (preservative) | .10 |
| Propylparaben (preservative) | .10 |
| De-ionized water | 96.75 |

The iota, kappa, and lambda polysaccharides are added and admixed under agitation to the water at room temperature. The pH of the resulting aqueous composition is adjusted to 4.0 by adding the citric acid. The preservatives are added while the aqueous composition is stirred. Care is taken to avoid entrainment of air when the aqueous composition is stirred or otherwise agitated. The resulting personal lubricant gel has a viscosity of 12,000 centipoise. A quantity of the lubricant gel is charged in a container that permits a selected metered amount of the gel to be dispensed from the container on multiple occasions. The container selected is configured to dispense 1.0 mL of the gel each time the container is utilized. The container can be operated to deliver the exact amount of lubricant (dosage) prescribed. Once such container is produced by Mega Pumps L.P. of 611 Industrial Way West, Eatontwon, NJ 07724. Any desired container can be utilized to dispense a metered amount of lubricant gel. The combination of a lubricant gel in a container that permits dispensation of metered amounts of the gel is an important feature of the invention because conventional lubricants typically are found in tubes or other containers that do not dispense multiple metered doses of lubricant. A particular desired feature of a container produced by Mega Pumps is that the container can dispense gel when the container is in any orientation. Further, the container prevents the admixing of air with the gel when the gel is dispensed. The container is preferably designed such that the container can-preferably with only a single hand--be grasped and manipulated by a user to dispense a dose of lubricant gel from a nozzle on the container directly into a user's nose, by woman to dispense a dose of lubricant gel into her vagina, etc. The nozzle can be configured to be inserted a selected distance in a user's nose, etc. prior to operating the container to select a metered dose of gel. During intercourse, or during the movement of any object contacting the gel, the motion of the object and friction generated between the object and gel cause the gel to liquify and spread out over the contact surface areas to reduce the surface frictional coefficients and provide increased lubrication. Although the amount of gel utilized can be varied as desired by a user, during sexual intercourse it is preferred that a sufficient dosage of lubricant gel be dispensed to introduce at least from 0.5 to 3.0 grams, preferably 1.0 to 2.0 grams, of carrageenan in the woman's vagina. This quantity of carrageenan is believed to facilitate lubrication and facilitates the inhibition of microphage movement. The thixotropic nature of the lubricant gel of the invention enables the gel to be applied one hour or more prior to sexual intercourse because the gel retains its viscosity and tends to maintain its position on epithelial tissue for an extended period of time, typically twenty-four to forty-eight hours, prior to any intercourse or prior to the gel's viscosity being decreased when the gel is contacted by a moving object.

### EXAMPLE II

Example I is repeated except that instead of 2% by weight of iota polysaccharide and 0.5% of lambda polysaccharide being utilized, 1.5% by weight of iota polysaccharide is utilized and 1% by weight of lambda polysaccharide is utilized. Similar results are obtained.

### EXAMPLE III

Example I is repeated except that instead of 96.75% by weight of water being utilized, 90% by weight of water is utilized and 6.75% by weight of glycerin is utilized. Similar results are obtained.

### EXAMPLE IV

Example I is repeated except that instead of 2% by weight of iota polysaccharide, 0.5% by weight of iota polysaccharide, and 0.5% by weight of kappa polysaccharide being utilized, and 3.0% by weight of iota polysaccharide is utilized. Similar results are obtained.

### EXAMPLE V

Example I is repeated except that instead of 0.05% by weight of citric acid being utilized, 0.05% of sodium hydroxide is utilized to adjust the pH to 8.0 instead of 4.0. Similar results are obtained.

### EXAMPLE VI

2.5 mL of the gel of EXAMPLE I is dispensed into a woman's vagina twenty-four hours prior to intercourse. Twenty-four hours later, just prior to intercourse, the gel is still present in the vagina and has retained its thixotropic characteristic.

### EXAMPLE VII

Example VI is repeated except that the gel is dispensed forty-eight hours prior to intercourse. Forty-eight hours later, just prior to intercourse, the gel is still present in the vagina and has retained its thixotropic characteristic.

### EXAMPLE VIII

Example 1 is repeated except that the gel is dispensed one hour prior to intercourse. One hour later, just prior to intercourse, the gel is still present in the vagina and has retained its thixotropic characteristic.

The iota, kappa, and lambda polysaccharides are sensitive to cations like sodium, potassium, and magnesium. The sodium cation increases the viscosity of the polysaccharides. It is therefore, in one embodiment of the invention, preferred to utilize a composition or component that functions as a fragrance and preservative and that includes a sodium, potassium, and/or magnesium ion. The concentration of the fragrance-preservative-ion composition in the finished product is in the range of 0.01 % to 0.15% by weight. One such preferred composition comprises sodium phytate.

In another important embodiment of the invention, production of the personal lubricant of the invention is accomplished by pre-mixing the iota, kappa, and/or lambda polysaccharide(s) with glycerin to form a pre-mix. The pre-mix is then combined with water. This is important because if the polysaccharide(s) is mixed directly with water, it tends to ball up, requiring the use of a homogenizer and an extended mixing time. The premix of glycerin and polysaccharide typically is mixed from 15 to 30 minutes, after which the premix is added to water. The premix includes from 60 to 90%, preferably from 70 to 85%, most preferably from 75 to 84% by weight glycerin, with the remainder of the premix comprising the polysaccharide(s). It is possible for the pre-mix to include up to 60% by weight polysaccharide(s), but in such a case the pre-mix must be added to water within five minutes. If the pre-mix includes from 20 to 25% by weight polysaccharide(s), there is a longer window, typically about twenty-five minutes, in which the glycerin-polysaccharide pre-mix must be added to water to avoid the polysaccharide absorbing all the water from the glycerin and turning the glycerin into a hard rock-like composition. When the pre-mix is combined with water to form a batch, the batch contains from 5% to 30% by weight pre-mix, preferably 10 to 28% premix, and most preferably 12 to 25% by weight pre-mix, with the remainder of the batch being water and small concentrations of fragrance, preservative(s), pH adjusting composition(s), and other desired additives. Citric acid can be added to adjust the pH to a preferred range of 4.12 to 4.25, although the pH can vary as desired.

When the water and premix are admixed in a tank, the temperature of the water is in the range of 45 C to 80 C, preferably 50 C to 70 C, and most preferably 55 C to 60C.

The glycerin that is preferably utilized is 97.5% glycerin, with the remainder of the glycerin being water. Some "glycerin" compositions are 60% to 70% by weight glycerin, with the remainder being water. Using glycerin compositions that are only 60% to 70% glycerin tends to defeat the purpose of first mixing the polysaccharides with glycerin because the polysaccharides will tend to clump into balls when they contact the water in the glycerin. Glycerin compositions that are at least 90% by weight glycerin are preferred in the practice of the invention because the polysaccharides tend during mixing to disperse uniformly in such glycerin compositions and tend not to clump.

Having described our invention in such terms as to enable those of skill in the art to make and practice it, and having described the presently preferred embodiments thereof, the invention is defined by the appended claims.

## Claims

1. A personal lubricant composition comprising a thixotropic gel
(a) having a viscosity in the range of 1,000 to 80,000 centipoise;
(b) having a pH in the range of 2.6 to 10; and
(c) comprising naturally occurring compositions including
(i) a high molecular weight, thixotropic, gel-forming, naturally-occurring polysaccharide extracted from algae and composed of repeating sulfated and non-sulfated galactose and 3,6 anhydrogalactose (3,6-AG) units; and,
(ii) water.

2. A method of lubricating mucosal tissue in the body to facilitate movement of a selected object over the tissue, comprising the steps of
(a) providing a container of a personal lubricant thixotropic gel to dispense multiple equivalent doses of said gel, said gel having a viscosity in the range of 1,000 to 80,000 centipoise, having a pH in the range of 2.6 to 10, and comprising water and a high molecular weight, thixotropic, gel-forming, naturally-occurring polysaccharide extracted from algae and composed of repeating sulfated and un-sulfated galactose and 3,6 anhydrogalactose (3,6-AG) units;
(b) applying to said mucosal tissue with said container at least two equivalent doses of said gel;
(c) moving the selected object over the mucosal tissue and gel to reduce the viscosity of the thixotropic gel and facilitate movement of the select object over the mucosal tissue.

3. A method of lubricating mucosal tissue in the body to facilitate movement of a selected object over the tissue during sexual intercourse, comprising the steps of
(a) providing a container of a personal lubricant thixotropic gel to dispense multiple equivalent doses of said gel, said gel having a viscosity in the range of 1,000 to 80,000 centipoise, having a pH in the range of 2.6 to 10, and comprising water and a high molecular weight, thixotropic, gel-forming, naturally-occurring polysaccharide extracted from algae and composed of repeating sulfated and un-sulfated galactose and 3,6 anhydrogalactose (3,6-AG) units, said gel produced by forming a premix of glycerin and polysaccharide and then combining said premix with water;
(b) applying at least one dose of said gel to said mucosal tissue with said container;
(c) moving the selected object over the mucosal tissue and gel during sexual intercourse to reduce the viscosity of the thixotropic gel and facilitate movement of the select object over the mucosal tissue.

4. The method of Claim 3 wherein in step (b) said dose of gel is applied at least one hour prior to step (c), and said gel maintains for said one hour prior to step (c) the position of the gel on said mucosal tissue and optionally wherein in step (b) said dose of gel is applied from one to twenty-four hours prior to step (c), and said gel maintains for said one to twenty-four hours prior to step (c) the position of the gel on said mucosal tissue.

5. The method of Claim 3 wherein in step (b) said dose of gel is applied from one to forty-eight hours prior to step (c), and said gel maintains for said one to forty-eight hours prior to step (c) the position of the gel on said mucosal tissue; and optionally wherein in step (b) said dose of gel is applied at least twenty-four hours prior to step (c), and said gel maintains for twenty-four hours prior to step (c) the position of the gel on said mucosal tissue.

6. The method of Claim 3 wherein in step (b) said dose of gel is applied at least forty-eight hours prior to step (c), and said gel maintains for forty-eight hours prior to step (c) the position of the gel on said mucosal tissue.

7. A topical composition for application to at least one of a pair consisting of epidermal tissue and mucosal tissue and comprising a thixotropic gel
(a) having a viscosity in the range of 400 to 80,000 centipoise;
(b) having a pH in the range of 2.6 to 10; and,
(c) comprising naturally occurring compositions including
(i) a high molecular weight, thixotropic, gel forming, naturally-occurring polysaccharide extracted from algae and composed of repeating sulfated and non-sulfated galactose and 3,6 anhydrogalactose (3,6-AG) units; and,
(ii) water.

8. The composition of Claim 9 including an effective amount of at least one selected auxiliary composition to contact the tissue and optionally wherein said selected auxiliary composition is selected from a group consisting of peptides, vitamins, minerals, microbicides, medicants, pain relief agents, emollients, exfoliants, surfactants, fragrances, antiperspirants, flavors, and skin colorants.

9. A method for massaging epidermal tissue, comprising the steps of
(a) providing a topical composition comprising a thixotropic gel
(i) having a viscosity in the range of 400 to 80,000 centipoise;
(ii) having a pH in the range of 2.6 to 10; and,
(iii) comprising naturally occurring compositions including
a high molecular weight, thixotropic, gel forming, naturally-occurring polysaccharide extracted from algae and composed of repeating sulfated and non-sulfated galactose and 3,6 anhydrogalactose (3,6-AG) units; and,
water; and,
(b) massaging the epidermal tissue with said topical composition.

10. A method of lubricating at least one of a pair comprising epidermal tissue and mucosal tissue, comprising the steps of
(a) providing a topical composition comprising a thixotropic gel
(i) having a viscosity in the range of 400 to 80,000 centipoise;
(ii) having a pH in the range of 2.6 to 10; and,
(iii) comprising naturally occurring compositions including
a high molecular weight, thixotropic, gel forming, naturally-occurring polysaccharide extracted from algae and composed of repeating sulfated and non-sulfated galactose and 3,6 anhydrogalactose (3,6-AG) units; and,
water; and,
(b) applying said topical composition to said tissue.
